# EUROPÄISCHE PATENTANMELDUNG

(11) **EP 3 903 749 A1**
(43) Veröffentlichungstag der Anmeldung: **03.11.2021**
(21) Anmeldenummer: 20172275.8
(22) Anmeldetag: 30.04.2020
(51) Int. Cl.: A61F 9/007

(54) **GLAUKOM STENT MIT KONTROLLIERTER WIRKSTOFFFREISETZUNG**

(71) Anmelder: KreCo Kreiner Consulting Gesellschaft für wissenschaftliches-technisches Projektmanagement mbH, 81541 München (DE)
(72) Erfinder: Kreiner, Hans Jürg, 81541 München (DE)
(74) Vertreter: Ter Meer Steinmeister & Partner

(57) **Zusammenfassung**

Die vorliegende Erfindung betrifft einen Glaukom Stent (100) umfassend einen überwiegend entlang einer Längsachse ausgedehnten Hohlzylinder, der abgeschrägte Enden (1) mit geschwungenem Profil aufweist. Des Weiteren gibt die vorliegende Erfindung ein Verfahren zur Herstellung eines solchen Glaukom Stents an.

## Beschreibung

Die vorliegende Erfindung betrifft einen Glaukom Stent umfassend einen überwiegend entlang einer Längsachse ausgedehnten Hohlzylinder, der abgeschrägte Enden mit geschwungenem Profil aufweist. Des Weiteren gibt die vorliegende Erfindung ein Verfahren zur Herstellung eines solchen Glaukom Stents an.

Glaukom (umgangssprachlich: Grüner Star) ist ein Sammelbegriff für eine Gruppe von Erkrankungen, die sich hinsichtlich ihrer Pathophysiologie, der Symptome sowie deren Behandlung unterscheiden. Gemeinsam ist ihnen, dass es sich um eine fortschreitende, neurodegenerative Erkrankung handelt, bei der es zu einem Verlust an retinalen Ganglienzellen und einer zunehmenden Aushöhlung des Sehnervenkopfs kommt. Dies macht sich in einem eingeschränkten Gesichtsfeld bemerkbar und kann schlussendlich zur Erblindung führen.

Obwohl das Fortschreiten der Krankheit durch geeignete Therapien aufgehalten werden kann, kam es im Jahr 2010 weltweit noch zur Erblindung von 2,1 Millionen Menschen durch eine Glaukomerkrankung (Bourne RR et al., PLoS One; 2016; 11(10), DOI:10.1371). Auch in Westeuropa ist das Glaukom nach der altersbedingten Makuladegeneration der zweithäufigste Grund für eine irreversible Erblindung (Bourne RRA et al., Br J Ophthalmol; 2018; 102(5); pp. 575-85).

Die in Westeuropa am meisten verbreitete Form des Glaukoms ist das sogenannte "Offenwinkelglaukom". Bei dieser Form der Erkrankung kommt es meist zu einer Erhöhung des Augeninnendruckes durch ein Ungleichgewicht zwischen der Menge an Kammerwasser, die produziert wird, und der Menge an Kammerwasser, die abfließen kann. Für dieses Ungleichgewicht ist beim primären Offenwinkelglaukom überwiegend ein pathologisch erhöhter Abflusswiderstand im Trabekelmaschenwerk ursächlich.

Das oberste Ziel bei der Behandlung des Offenwinkelglaukoms besteht daher in der Senkung und dauerhaften Regulierung Augeninnendruckes. Dies kann mittels verschiedener Therapieansätze erreicht werden.

Oft wird zunächst versucht, den Augeninnendruck medikamentös mit Hilfe von Augentropfen zu reduzieren und einzustellen. Verschiedene augendrucksenkende Wirkstoffe sind bekannt. Die Wirkstoffe aus der Klasse der β-Blocker und Carboanhydrasehemmer tragen beispielsweise zu einer verminderten der Kammerwasserproduktion bei, während Cholinergika, Parasympathomimetika, Prostaglandinderivate den Kammerwasserabfluss am Trabekelmaschenwerk und/oder den uveoskleralen Abfluss verbessern.

Zeigt der medikamentöse Therapieansatz nicht den gewünschten Effekt oder fehlt die nötige Patientencompliance für eine regelmäßige Anwendung, gibt es die Möglichkeit, verschiedene chirurgische Eingriffe vornehmen zu lassen.

Die älteste und noch immer am häufigsten durchgeführte Operation, um den Augeninnendruck dauerhaft zu senken, ist die Trabekulektomie. Bei dieser wird unter der Bindehaut ein neue, künstliche Abflussöffnung für das Kammerwasser geschaffen. Im Einzelnen wird hierbei ein kleines Stückchen der Sklera (Lederhaut) eingeschnitten, angehoben und zu einem "Deckel" präpariert. Um den Abfluss weiter zu erleichtern, wird auch ein kleiner Anteil der Iris entfernt (Iridektomie). So kann das Kammerwasser von der hinteren in die vordere Augenkammer fließen, von wo es in die Bindehaut sickert und dort von den Gefäßen abtransportiert wird. Das Abflussloch mit dem Skleradeckel funktioniert wie eine Art Druckventil. An der Stelle, wo sich das Augenwasser vor dem Abtransport sammelt, entsteht eine Vorwölbung, das sogenannte Filter- oder Sickerkissen.

Die Trabekulektomie ist jedoch keine uneingeschränkt empfehlenswerte Behandlungsmethode und führt nicht immer zum Erfolg.

Beispielsweise wird oft beobachtet, dass es durch den natürlichen Heilungsprozess zur Narbenbildung am Filterkissen und zu einer sogenannten Abkapselung kommt. Diese Abkapselung führt zu einem erneuten Ansteigen des Augeninnendrucks. Als Gegenmaßnahme können schon während der OP und/oder postoperativ Wirkstoffe (z.B Mitomycin-C und 5-Fluorouracil) appliziert werden, jedoch kann auch eine Verabreichung dieser Wirkstoffe eine Vernarbung nicht sicher verhindern.

Aber auch bei erfolgreicher Trabekulektomie sind regelmäßige Kontrollen erforderlich, denn das Filterkissen kann nach Mitomycin-C-Anwendung noch nach Jahren Mikroläsionen entwickeln, die zu Eintrittspforten für Bakterien werden und dann eine Durchwanderungsendophthalmitis zur Folge haben.

Ein weiterer Nachteil bei der Trabekulektomie ist, dass sich das Risiko zur Ausbildung eines grauen Stares (Linsentrübung, Katarakt) erhöht, weil das Kammerwasser nicht mehr wie zuvor die Linse umspült.

Eine Alternative zur Trabekulektomie ist ein minimalinvasiver Eingriff, z.B. die Implantation eines Glaukom Stents in den Schlemm'schen Kanal, der den natürlichen Abfluss des Kammerwassers unterstützt. Diese Methode ist vorteilhaft, da keine Eröffnung der Bindehaut nötig ist, der Eingriff kein Fremdkörpergefühl hinterlässt und gut mit einer Operation des grauen Stars kombinierbar ist.

Glaukom Stents, die bisher bei dieser Methode eingesetzt werden, können beispielsweise von der Firma Glaukos bezogen werden. Der iStent® der Firma Glaukos ist ein 1-teiliges L-förmiges Titanimplantat, dessen Länge etwa 1 mm und dessen Außendurchmesser ca. 180 µm beträgt. Das rechtwinklig dazu verlaufende kurze Ende ragt mit seinem Lumen in die Vorderkammer und schafft eine permanente Verbindung zwischen vorderer Augenkammer und Schlemm-Kanal.

Dennoch treten auch hier Komplikationen auf: Neben einer Fehlpositionierung des Stents ist die häufigste Ursache für Komplikationen die Obstruktion des Stentlumens (Wellik SR et al.; Clin Ophthalmol. 2015; 9, pp. 677-684). Daher besteht auch bei diesem Therapieansatz noch Verbesserungsbedarf.

Ein weiterer verbesserungswürdiger Aspekt ist die Beschichtung der Glaukom Stents. Der iStent® weist standardäßig eine Heparinbeschichtung auf. Indes ist der iStent® nicht mit einer äußeren pharmakonhaltigen Beschichtung erhältlich, die eine kontrollierte lokale Wirkstofffreisetzung über einen langen Zeitraum ermöglicht und die Senkung des Augeninnendrucks unterstützen würde. Der iStent® schöpft daher längst nicht alle Möglichkeiten zur effektiven Senkung des Augeninnendrucks aus.

Diese Nachteile sollten durch die vorliegende Erfindung überwunden werden. Ausgehend vom oben beschriebenen Stand der Technik bestand die der vorliegenden Erfindung zugrundeliegende Aufgabe in der Bereitstellung eines verbesserten Glaukom Stents. Wichtig war insbesondere, einen Glaukom Stent zu entwickeln, der noch weniger anfällig für eine Okklusion des Stentlumens ist als bekannte Stents. Daneben sollte der neue Glaukom Stent auch zu einer effektiveren und nachhaltigeren Reduzierung des Augeninnendrucks führen. Gleichzeitig sollte auch ein Verfahren angegeben werden, mit dem der neue Glaukom Stent hergestellt werden kann.

Diese Aufgaben wurden überraschend durch den Glaukom Stent gemäß Anspruch 1 und das Verfahren gemäß Anspruch 13 gelöst. Bevorzugte Ausführungsformen gehen aus den abhängigen Ansprüchen hervor.

Erfindungsgemäß wird ein Glaukom Stent umfassend einen überwiegend entlang einer Längsachse ausgedehnten Hohlzylinder, welcher einen Durchmesser von 0,20 bis 2,50 mm und abgeschrägte Enden mit geschwungenem Profil aufweist, bereitgestellt.

Dieser Glaukom Stent eignet sich aufgrund der Abmessungen und Formgebung in besonderem Maße zur Implantation in den Kammerwinkel des Auges.

Die Ausgestaltung der Enden, die wie löffelförmige Ausläufer anmuten, ist in zweierlei Hinsicht vorteilhaft: Einerseits erlaubt sie ein gutes, widerstandsarmes und schmerzfreies Eindringen in das Gewebe. Andererseits wird das Anhaften von Zellen erschwert und ein Verschluss des Stentlumens verhindert.

Ein "Hohlzylinder" im Sinne der vorliegenden Erfindung ist ein Zylinder, der offene Enden und einen Durchgang besitzt und somit einem Rohr bzw. Röhrchen ähnelt.

Die Formulierung "überwiegend entlang einer Längsachse ausgedehnter Hohlzylinder" bedeutet, dass der Hohlzylinder höchstens leicht in eine Richtung gekrümmt ist, um sich z.B. an die Form des Schlemm-Kanals anzupassen. Leicht gekrümmt bedeutet, dass der Krümmungsradius des Hohlzylinders mindestens 1/3 der gesamten Länge des Hohlzylinders entspricht.

Ob die abgeschrägten Enden des Hohlzylinders ein geschwungenes Profil aufweisen, kann am einfachsten an einer Projektion des Hohlzylinders auf eine Ebene, die von der Längsachse und einer senkrecht zur Längsachse verlaufenden Achse aufgespannt wird, festgestellt werden. Das heißt, dass beispielsweise am sogenannten Seitenriss des Stents untersucht wird, ob der erfindungsgemäße Hohlzylinder abgeschrägte Enden mit geschwungenem Profil aufweist.

In einer bevorzugten Ausführungsform besteht der Stent zu mindestens 60 Gew.-%, bevorzugt zu mindestens 65 Gew.-%, besonders bevorzugt zu mindestens 70 Gew.-% aus dem Hohlzylinder. Dadurch wird die Hauptfunktion des Stents gewahrt, nämlich einen gut durchlässigen Kanal von der vorderen Augenkammer zum distalen Gewebe zu schaffen und den Abfluss von Kammerwasser zu erleichtern.

Des Weiteren weisen die abgeschrägten Enden des Stents bevorzugt ein stetiges Profil auf. Besonders bevorzugt ist das Profil s-förmig geschwungen. Das s-förmig geschwungene Profil kann auch als sigmoidales Profil bezeichnet werden.

Aus fertigungstechnischer Sicht vorteilhaft ist, wenn der Hohlzylinder gerade und nicht gekrümmt ausgebildet ist. Weiterhin können an der Mantelfläche des Hohlzylinders Vorsprünge und/oder Widerhaken vorhanden sein. Es ist allerdings trotz vorhandener Vorsprünge und/oder Widerhaken bevorzugt, wenn der Stent an sich eine zweizählige Rotationssymmetrie um eine senkrecht zur Längsachse des Hohlzylinders verlaufende Achse aufweist. Eine Rotationssymmetrie um die Längsachse selbst besitzt der Stent selbst in dem Fall, in dem der Hohlzylinder keine Krümmung aufweist, nicht. Grund hierfür sind die nicht rotationssymmetrisch ausgebildeten abgeschrägten Enden des Hohlzylinders.

Es ist weiterhin bevorzugt, wenn der Durchmesser des Hohlzylinders noch geringer als die bereits zuvor angegebenen 0,20 bis 2,50 mm ist. Der Durchmesser des Hohlzylinders liegt daher bevorzugt in einem Bereich von 0,20 bis 1,20 mm, besonders bevorzugt in einem Bereich von 0,25 bis 0,90 mm, insbesondere in einem Bereich von 0,30 bis 0,70 mm. Die Wandstärke des Hohlzylinders ist gering und beträgt bevorzugt von 0,05 bis 0,2 mm, besonders bevorzugt von 0,05 bis 0,1 mm. Mit diesen Dimensionen kann gewährleistet werden, dass das Stentlumen ausreichend groß ist und einen Durchmesser von mindestens 100 µm, bevorzugt von mindestens 200 µm, aufweist.

Entlang der Längsachse weist der Stent bevorzugt eine Länge von 2,5 mm bis 10 mm, besonders bevorzugt von 2,5 mm bis 7,0 mm, ganz besonders bevorzugt von 2,5 bis 5,0 mm, auf. Mit diesen Abmessungen gehört der Glaukom Stent zu den kleinsten Implantaten weltweit und es kann sichergestellt werden, dass der Stent nach seiner Implantation nicht als störend wahrgenommen wird.

In einer Ausführungsform umfasst der Stent ferner ein Ventil. Dieses kann ein druckgesteuertes mikromechanisches Ventil sein, welches einen Öffnungsdruck im Bereich von 0,5 mmHg und 4,0 mmHg aufweist. Dadurch dass ein solches Ventil in den Stent integriert ist, können Flüssigkeiten nur in eine Richtung transportiert werden, ein Durchfluss in die entgegengesetzte Richtung ist gesperrt. Zudem wird dem Abtransport von Kammerwasser wenig Widerstand entgegengesetzt, sobald der Augeninnendruck über den Öffnungsdruck des Ventils steigt. Eine effektive Einstellung des Augeninnendrucks auf den gewünschten Druck wird möglich.

Ferner ist bevorzugt, wenn der Stent zu mindestens 90 Gew.-% aus einem gemäß der DIN EN ISO 10993-1:2010-04 biokompatiblen Material besteht. Das biokompatible Material ist dabei bevorzugt ein Polymer. Das Polymer weist vorteilhafterweise eine gewisse thermische Stabilität auf und lässt sich mittels Extrusion verarbeiten. Besonders bevorzugt ist das Polymer ausgewählt aus der Gruppe bestehend aus Polymethylmethacrylat (PMMA), Polyvinylpyrrolidonen (PVP), Polytetrafluorethylen (PTFE), Polyolefinen, Silikonen, Polyvinylalkoholen (PVA), Polycarbonaten, PEEK, sowie Copolymeren und Blends hiervon. Vorzugsweise ist das Polymer aus der Gruppe bestehend aus Polycarbonat-basierten Silikonelastomeren, z.B. ChronoSil® (Firma AdvanSource Biomaterials), Polyether-basierten thermoplastischen Polyurethanen, z.B. Pellethanen® (Firma Lubrizol), Poly(L-lactid) und Kombinationen hiervon ausgewählt.

ChronoSil® ist ein biokompatibles Material, welches die vorteilhaften Eigenschaften von Polycarbonat-basierten Urethanen (gute Zugfestigkeit und chemische Beständigkeit) und Silikonen (Dehnverhalten und Elastizität) vereint. Pellethane® sind dafür bekannt, dass sie ideal für eine Reihe verschiedener medizinischer Anwendungen geeignet sind, zum Beispiel zur Herstellung medizinischer Schläuche und Katheter.

Auf den löffelförmigen Innenseiten der geschwungen auslaufenden Enden des Hohlzylinders kann jedoch eine Schicht zytotoxischen Materials aufgebracht sein. Bevorzugt ist diese Schicht eine dünne Schicht, insbesondere eine dünne Schicht umfassend Kupfer, Silber, Zink, Magnesium oder Kombinationen oder Legierungen hiervon. Bevorzugt weist die Schicht eine Schichtdicke von 5 nm bis 20 µm, insbesondere von 20 nm bis 5 µm auf. Die aufgezählten Metalle wirken antiproliferativ und tragen somit weiter dazu bei, dass eine Anlagerung von Zellen und ein Verschluss des Stentlumen verhindert werden kann.

Zudem kann auf der Innen- und/oder Außenseite der Mantelfläche des Hohlkörpers, besonders bevorzugt auf der Außenseite der Mantelfläche, eine Beschichtung aufgebracht sein. Diese Beschichtung umfasst bevorzugt mindestens einen Wirkstoff und mindestens ein Beschichtungsmaterial, das bioabbaubar und gemäß der DIN EN ISO 10993-1:2010-04 biokompatibel ist.

Der mindestens eine Wirkstoff ist bevorzugt ein antiglaumatöser Wirkstoff, besonders bevorzugt ein Wirkstoff, der aus der Gruppe bestehend aus Betablockern, Prostaglandinen, Parasympathomimetika, Sympathomimetika, Carboanhydrasehemmern, Antioxidantien und Kombinationen hiervon ausgewählt ist.

Die Betablocker sind dabei vorzugsweise ausgewählt aus der Gruppe bestehend aus Timolol, Propanolol, Betaxolol, Levobunolol, Metipranolol, Carteolol, Pindolol, Befunolol sowie deren Salzen, Analoga, Derivaten und Kombinationen hiervon. Die Vertreter der Prostaglandine sind bevorzugt Bimatoprost, Latanoprost, Travoprost und/oder deren Salze, Analoga, Derivate und Kombinationen hiervon. Als Parasympathomimetika werden bevorzugt Pilocarpin, Carbachol und deren Salze, Analoga, Derivate und Kombinationen hiervon verwendet. Die Sympathomimetika können aus Brimonidin, Clonidin, Apraclonidin, Dipivefrin sowie aus deren Salzen, Analoga, Derivaten und Kombinationen hiervon bestehen. Die Carboanhydrasehemmer sind vorteilhafterweise ausgewählt aus der Gruppe bestehend aus Dorzolamid, Brinzolamid sowie deren Salzen, Analoga, Derivaten und Kombinationen hiervon. Für die Klasse der Antioxidantien wird bevorzugt Resveratrol und dessen Salze, Analoga und Derivate eingesetzt.

Die Wirkstoffe liegen bevorzugt als Partikel vor. Die Größe dieser Partikel richtet sich nach der Freisetzungsgeschwindigkeit, die in vivo angestrebt wird. Die absolute Menge an Wirkstoff wird so gewählt, dass nach Implantation des Stents, die empfohlene Maximalkonzentrationen nicht überschritten wird.

Das mindestens eine Beschichtungsmaterial ist bevorzugt ein hydrolysierbares und/oder enzymatisch spaltbares Makromolekül. Das Makromolekül ist bevorzugt ein hydrophiles Polymer. Es kann sowohl aus der Gruppe der synthetischen als auch aus der Gruppe der natürlichen Makromoleküle ausgewählt sein. Zur Gruppe der natürlichen Makromoleküle gehören Polysaccharide wie zum Beispiel Cellulose, Hydroxy-(ethylpropyl)-cellulose, Carmellose und Hypromellose. Besonders bevorzugt ist das Beschichtungsmaterial aber ausgewählt aus der Gruppe bestehend aus Hyaluronsäure, quervernetzter Hyaluronsäure, Alginat, Polyglykolsäure, Propylenglykolalginat, Polymilchsäure, Gelatine, Collagen, Polyethylenvinylacetat, Polycaprolacton sowie Copolymeren und Blends hiervon. Die quervernetzte Hyaluronsäure kann beispielsweise durch Quervernetzung einer Hyaluronsäure mit einem niedrigen oder mittleren gemittelten Molekulargewicht von 0,8 bis 1,0 Millionen Dalton mit BDDE oder Epichlorhydrin gewonnen werden. Besonders bevorzugt liegt das Beschichtungsmaterial in Form von Fäden und/oder Filamenten vor, wobei die Fäden und/oder Filamente besonders bevorzugt mehrfach um die Außenseite der Mantelfläche des Hohlzylinders gewickelt sind.

Die Beschichtung umfasst bevorzugt eine innere Schale und eine äußere Schale, wobei die innere Schale den mindestens einen Wirkstoff enthält und die äußere Schale im Wesentlichen aus dem mindestens einen bioabbaubaren Material besteht. Während die äußere Schale keinen Wirkstoff enthält und die innere Schale für eine gewisse Zeit "abschirmt", kann die innere Schale neben dem Wirkstoff auch das mindestens eine bioabbaubare Material enthalten. In diesem Fall, das heißt, wenn die innere Schale bioabbaubares Material enthält, dann dient dieses bioabbaubare Material gewissermaßen als Trägermaterial für den Wirkstoff. Beispielsweise kann die innere Schale der Beschichtung aus quervernetzter Hyaluronsäure bestehen, die mit Timolol-Maleat beladen ist, während die äußere Schale nur aus quervernetzter Hyaluronsäure ohne Wirkstoff besteht.

Unter physiologischen Bedingungen bildet das bioabbaubare Material bevorzugt ein Hydrogel aus. Dies geschieht, indem wässriges Medium aus der Umgebung in das poröse Netzwerk eingelagert wird und dieses aufquillt.

Es ist besonders vorteilhaft, wenn die Schichtdicke der äußeren Beschichtungsschale entlang der Längsachse des Hohlzylinders variiert (entweder annähernd kontinuierlich oder stufenförmig). Dies ermöglicht es, den Wirkstoff mit unterschiedlicher Verzögerung nach Einsetzen des Stents in den Kammerwinkel freizusetzen. Die innere Schale, welche den Wirkstoff enthält, wird beispielsweise durch einen fortschreitenden Abbau des Beschichtungsmaterials durch Hydrolyse oder enzymatische Spaltung nur abschnittsweise freigelegt. Dementsprechend können immer nur Teil des Wirkstoffes verstoffwechselt werden. Bevorzugt variiert die Dicke der Beschichtung in einem Bereich von 0,01 mm bis 0,02 mm. Die Beschichtungsdicke ist in dem Fall, in dem das Beschichtungsmaterial in Form von Fäden und/oder Filamenten vorliegt, beispielsweise proportional zur Anzahl der Wicklungslagen.

Ein weiterer Aspekt der Erfindung besteht in der Bereitstellung eines Verfahrens zur Herstellung eines Glaukom Stents, bei dem ein überwiegend entlang einer Längsachse ausgedehnter Hohlzylinder, welcher einen Durchmesser von 0,20 bis 2,50 mm und abgeschrägte Enden mit geschwungenem Profil aufweist, mit Hilfe eines Formungsverfahrens hergestellt wird. Das Formungsverfahren umfasst vorteilhafterweise einen Extrusionsschritt sowie optional einen Laser-Schneideprozess. Denkbar sind aber auch andere Formungsverfahren wie additive Fertigungstechniken oder andere dem Fachmann bekannte Techniken.

Nach Herstellung des Hohlzylinders werden die löffelförmigen Innenseiten der geschwungen auslaufenden Enden des Glaukom Stents bevorzugt mit zytotoxischem Material besputtert, insbesondere mit einem zytotoxischen Material ausgewählt aus der Gruppe bestehend aus Kupfer, Silber, Zink, Magnesium oder Kombinationen oder Legierungen hiervon.

Abgesehen davon kann ein weiterer Verfahrensschritt vorgesehen sein, bei dem die Außenseite der Mantelfläche des Hohlkörpers mit mindestens einem bioabbaubaren Material und mindestens einem Wirkstoff beschichtet wird, wobei das Beschichten bevorzugt Schritte a) und b) und optional weitere Schritte c) und d) umfasst:
a) Bereitstellen einer Wirkstoff-Lösung umfassend ein Lösungsmittel, sowie das mindestens eine bioabbaubare Material und den mindestens einen Wirkstoff,
b) Elektrospinnen der Wirkstoff-Lösung zu wirkstoffbeladenen Fäden, die um die Außenseite der Mantelfläche des Hohlkörpers gewickelt werden, um eine Wirkstoffbeschichtung zu erhalten,
c) Bereitstellen einer weiteren Lösung, welche im Wesentlichen aus einem Lösungsmittel, optional Spinnhilfsmitteln, sowie dem mindestens einen bioabbaubaren Material besteht, und
d) Elektrospinnen der weiteren Lösung zu Fäden, die um die Außenseite der Wirkstoffbeschichtung gewickelt werden, um eine Beschichtung zu erhalten, die eine äußere Schale umfasst, welche im Wesentlichen aus dem mindestens einen bioabbaubaren Material besteht.

Das Lösungsmittel, welches zum Herstellen der Spinnlösungen (der Wirkstofflösung in Schritt a und der weiteren Lösung in Schritt c) verwendet wird, ist bevorzugt ein Alkohol, z.B. Ethanol.

Bevorzugte Ausführungsformen der Erfindung werden unter Bezugnahme auf die nachfolgenden Beispiele und Figuren näher erläutert, ohne die Erfindung darauf beschränken zu wollen.
Figur 1 zeigt den erfindungsgemäßen Glaukom Stent aus einer Perspektive, die einen Blick in das Stentlumen ermöglicht.
Figur 2 zeigen den erfindungsgemäßen Glaukom Stent aus einer Seitenperspektive.
Figur 3 zeigt eine bevorzugte Ausführungsform des erfindungsgemäßen Glaukom Stents im Seitenriss, d.h. in der Ebene A-A, welche in Figur 1 eingezeichnet ist.

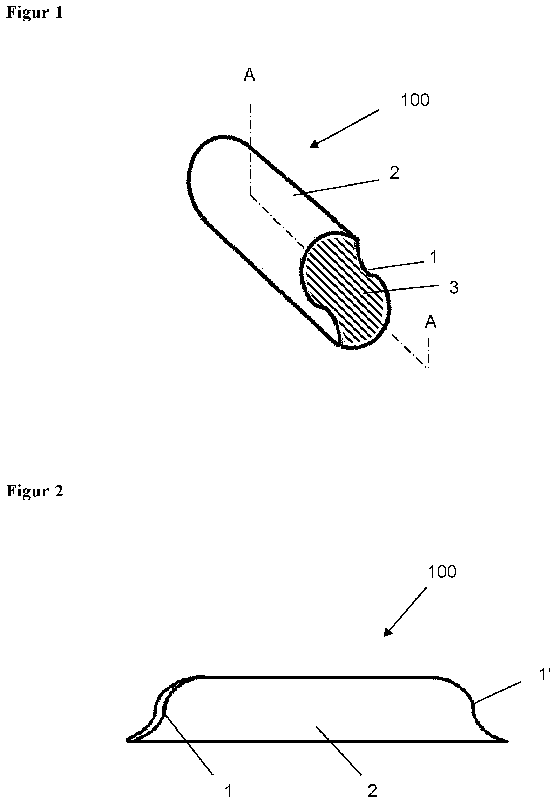

Der Glaukom Stent 100 in Fig. 1 besteht aus einem entlang einer Längsachse ausgedehnten Hohlzylinder, der abgeschrägte Enden 1 mit geschwungenem Profil aufweist. Die Mantelfläche 2 ist in der gezeigten Ausführungsform noch unbeschichtet. Die schraffierte Fläche 3 deutet den Eingang in das Lumen des Stents an. Durch das s-förmig geschwungene Profil des abgeschrägten Endes 1 erinnert der Eingang in das Lumen des Stents an eine Löffelform.

In Fig. 2 ist der Glaukom Stent 100 aus einer seitlichen Perspektive gezeigt. Es ist zu sehen, dass beide Enden des Hohlzylinders 1 und 1' abgeschrägt sind und ein geschwungenes s-förmiges Profil aufweisen. Auch hier ist die Mantelfläche 2 des Hohlzylinders ohne Beschichtung dargestellt.

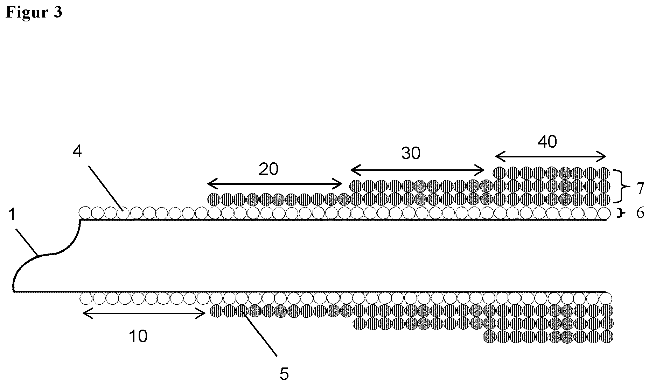

Fig. 3 zeigt hingegen eine exemplarische Beschichtung des Glaukom Stents 100. Im Seitenriss sieht man die innere Schale 6 der Beschichtung. Diese besteht in Fig. 3 aus einer Wicklung elektrogesponnener und mit dem Wirkstoff Timololmaleat beladener Hyaluronsäurefäden 4. Die innere Schale 6 liegt nur in dem Bereich 10 frei und ist ansonsten von einem Kontakt mit dem umgebenden Medium abgeschirmt. Diese Abschirmfunktion wird durch die äußere Schale 7 übernommen, welche durch Hyaluronsäurefäden (ohne Wirkstoffbeladung) 5 gebildet wird. Vorliegend ist dargestellt, dass abschnittsweise mehrere Lagen reiner Hyaluronsäurefäden 5 um die innere Schale 6 gewickelt werden. Im Bereich 20 schirmt nur eine Wicklungslage die innere Schale 6 von einem direkten Kontakt mit dem umgebenden Medium ab. Im Bereich 30 sind es zwei Wicklungslagen und im Bereich 40 drei Wicklungslagen, welche die innere Schale 6 schützen. Durch den hydrolytischen und/oder enzymatischen Abbau der Hyaluronsäure in der äußeren Schale 7 wird der Wirkstoff in der inneren Schale nach und nach zugänglich und kann kontrolliert und über einen langen Zeitraum hinweg freigesetzt werden.

## Patentansprüche

1. Glaukom Stent umfassend einen überwiegend entlang einer Längsachse ausgedehnten Hohlzylinder, welcher einen Durchmesser von 0,20 bis 2,50 mm und abgeschrägte Enden mit geschwungenem Profil aufweist.

2. Glaukom Stent gemäß Anspruch 1, wobei die abgeschrägten Enden ein s-förmig geschwungenes Profil aufweisen.

3. Glaukom Stent gemäß mindestens einem der vorherigen Ansprüche, wobei der Stent eine zweizählige Rotationssymmetrie um eine senkrecht zur Längsachse des Hohlzylinders verlaufende Achse aufweist, bevorzugt aber keine Rotationssymmetrie um die Längsachse besitzt.

4. Glaukom Stent gemäß mindestens einem der vorherigen Ansprüche, wobei der Hohlzylinder einen Durchmesser von 0,20 bis 1,20 mm, bevorzugt von 0,25 bis 0,90 mm, besonders bevorzugt von 0,30 bis 0,70 mm, und eine entlang der Längsachse gemessene Länge von 2,5 mm bis 10 mm, bevorzugt von 2,5 mm bis 7,0 mm, besonders bevorzugt von 2,5 bis 5,0 mm, aufweist.

5. Glaukom Stent gemäß mindestens einem der vorherigen Ansprüche, wobei der Stent weiterhin ein Ventil umfasst, bevorzugt ein druckgesteuertes mikromechanisches Ventil, besonders bevorzugt mit einem Öffnungsdruck im Bereich von 0,5 mmHg und 4,0 mmHg.

6. Glaukom Stent gemäß mindestens einem der vorherigen Ansprüche, wobei der Stent zu mindestens 90 Gew.-% aus einem gemäß der DIN EN ISO 10993-1:2010-04 biokompatiblen Material besteht, wobei das biokompatible Material bevorzugt ausgewählt ist aus der Gruppe bestehend aus Polymethylmethacrylat (PMMA), Polyvinylpyrrolidonen (PVP), Polytetrafluorethylen (PTFE), Polyolefinen, Silikonen, Polyvinylalkoholen (PVA), Polycarbonaten, PEEK, sowie Copolymeren und Blends hiervon, besonders bevorzugt aus der Gruppe bestehend aus Polycarbonat basierten Silikonelastomeren, z.B. ChronoSil®.

7. Glaukom Stent gemäß mindestens einem der vorherigen Ansprüche, wobei auf den löffelförmigen Innenseiten der geschwungen auslaufenden Enden eine Schicht zytotoxischen Materials aufgebracht ist, bevorzugt eine Schicht umfassend Kupfer, Silber, Zink, Magnesium oder Kombinationen oder Legierungen hiervon, wobei die Schicht zytotoxischen Materials besonders bevorzugt eine Schichtdicke von 5 nm bis 20 µm, insbesondere von 20 nm bis 5 µm aufweist.

8. Glaukom Stent gemäß mindestens einem der vorherigen Ansprüche, wobei auf der Außenseite der Mantelfläche des Hohlkörpers eine Beschichtung aufgebracht ist, bevorzugt eine Beschichtung umfassend mindestens einen Wirkstoff und mindestens ein Beschichtungsmaterial, das bioabbaubar und gemäß der DIN EN ISO 10993-1:2010-04 biokompatibel ist.

9. Glaukom Stent gemäß Anspruch 8, wobei der mindestens eine Wirkstoff bevorzugt aus der Gruppe bestehend aus Betablockern, Prostaglandinen, Parasympathomimetika, Sympathomimetika, Carboanhydrasehemmern, Antioxidantien und Kombinationen hiervon ausgewählt ist,
wobei die Betablocker besonders bevorzugt ausgewählt sind aus der Gruppe bestehend aus Timolol, Propanolol, Betaxolol, Levobunolol, Metipranolol, Carteolol, Pindolol, Befunolol und Kombinationen hiervon,
die Prostaglandine besonders bevorzugt ausgewählt sind aus der Gruppe bestehend aus Bimatoprost, Latanoprost, Travoprost und Kombinationen hiervon,
die Parasympathomimetika besonders bevorzugt ausgewählt sind aus Pilocarpin, Carbachol und Kombinationen hiervon,
die Sympathomimetika besonders bevorzugt ausgewählt sind aus Brimonidin, Clonidin, Apraclonidin, Dipivefrin und Kombinationen hiervon,
die Carboanhydrasehemmer besonders bevorzugt ausgewählt sind aus der Gruppe bestehend aus Dorzolamid, Brinzolamid und Kombinationen hiervon, die Antioxidantien besonders bevorzugt ausgewählt sind aus der Gruppe bestehend aus Resveratrol.

10. Glaukom Stent gemäß mindestens einem der Ansprüche 8 und 9, wobei das mindestens eine Beschichtungsmaterial ein hydrolysierbares und/oder enzymatisch spaltbares Makromolekül ist, welches bevorzugt aus der Gruppe bestehend aus Hyaluronsäure, quervernetzter Hyaluronsäure, Alginat, Polyglykolsäure, Propylenglykolalginat, Polymilchsäure, Gelatine, Collagen, Polyethylenvinylacetat, Polycaprolacton sowie Copolymeren und Blends hiervon ausgewählt ist.

11. Glaukom Stent gemäß mindestens einem der Ansprüche 8 bis 10, wobei die Beschichtung eine innere Schale und eine äußere Schale umfasst, wobei die innere Schale den mindestens einen Wirkstoff enthält und die äußere Schale im Wesentlichen aus dem mindestens einen bioabbaubaren Material besteht.

12. Glaukom Stent gemäß Anspruch 11, wobei die Schichtdicke der äußeren Schale entlang der Längsachse des Hohlzylinders variiert, und bevorzugt in einem Bereich von 0,01 mm bis 0,02 mm liegt.

13. Verfahren zur Herstellung eines Glaukom Stents, bei dem ein überwiegend entlang einer Längsachse ausgedehnter Hohlzylinder, welcher einen Durchmesser von 0,20 bis 2,50 mm und abgeschrägte Enden mit geschwungenem Profil aufweist, mit Hilfe eines Formungsverfahrens, vorzugsweise eines Extrusionsverfahrens, optional gefolgt von einem Zuschneiden mittels Laser, hergestellt wird.

14. Verfahren gemäß Anspruch 13, wobei die löffelförmigen Innenseiten der geschwungen auslaufenden Enden mit zytotoxischem Material besputtert werden.

15. Verfahren gemäß mindestens einem der Ansprüche 13 oder 14, wobei die Außenseite der Mantelfläche des Hohlkörpers mit mindestens einem bioabbaubaren Material und mindestens einem Wirkstoff beschichtet wird, wobei das Beschichten bevorzugt Schritte a) und b) und optional weitere Schritte c) und d) umfasst:
a) Bereitstellen einer Wirkstoff-Lösung umfassend ein Lösungsmittel, sowie das mindestens eine bioabbaubare Material und den mindestens einen Wirkstoff,
b) Elektrospinnen der Wirkstoff-Lösung zu wirkstoffbeladenen Fäden, die um die Außenseite der Mantelfläche des Hohlkörpers gewickelt werden, um eine Wirkstoffbeschichtung zu erhalten,
c) Bereitstellen einer weiteren Lösung, welche im Wesentlichen aus einem Lösungsmittel, optional Spinnhilfsmitteln, sowie dem mindestens einen bioabbaubaren Material besteht, und
d) Elektrospinnen der weiteren Lösung zu Fäden, die um die Außenseite der Wirkstoffbeschichtung gewickelt werden, um eine Beschichtung zu erhalten, die eine äußere Schale umfasst, welche im Wesentlichen aus dem mindestens einen bioabbaubaren Material besteht.
